# EUROPEAN PATENT SPECIFICATION

(11) **EP 1 141 310 B1**
(45) Date of publication and mention of the grant of the patent: **07.12.2005**
(21) Application number: 99966676.1
(22) Date of filing: 21.12.1999
(51) Int. Cl.: C12N 15/31, C12N 15/75, C12N 9/28, C12N 9/56, C07K 14/32, C12P 21/02

(54) **PRODUCTION OF PROTEINS IN GRAM-POSITIVE MICROORGANISMS**
PROTEINPRODUKTION IN GRAM-POSITIVEN MIKROORGANISMEN
PRODUCTION DE PROTEINES DANS DES MICRO-ORGANISMES GRAM-POSITIF

(30) Priority: 24.12.1998 GB 9828711
(43) Date of publication of application: 10.10.2001
(73) Proprietor: GENENCOR INTERNATIONAL, INC., Palo Alto, California 94304 (US)
(72) Inventor: DIAZ-TORRES, Maria, Palo Alto, CA 94304 (US); FERRARI, Eugenio, Palo Alto, CA 94304 (US)
(74) Representative: Brasnett, Adrian Hugh
(86) International application number: PCT/US1999/031010
(87) International publication number: WO 2000/039323

(56) References cited:
- US-A- 5 387 521
- PODBIELDSKI A ET AL.: "Molecular characterization of group A streptococcal (GAS) oligopeptide permease (opp) and its effect on cysteine protease production" MOLECULAR MICROBIOLOGY, vol. 21, no. 5, September 1996 (1996-09), pages 1087-1099, XP000906862
- PODBIELSKI A ET AL.: "The group A streptococcal dipeptide permease (Dpp) is involved in the uptake of essential amino acids and affects the expression of cysteine protease" MOLECULAR MICROBIOLOGY, vol. 28, no. 6, June 1998 (1998-06), pages 1323-1334, XP000906863
- LEDEAUX J R ET AL.: "Analysis of non-polar deletion mutations in the genes of the spo0K (opp) operon of Bacillus subtilis" FEMS MICROBIOLOGY LETTERS, vol. 153, no. 1, August 1997 (1997-08), pages 63-69, XP000914621 cited in the application
- WONG S L: "Advances in the use of Bacillus subtilis for the expression and secretion of heterologous proteins" CURRENT OPINION IN BIOTECHNOLOGY, vol. 6, no. 5, 1 October 1995 (1995-10-01), pages 517-522, XP000607468
- HENNER ET AL: 'Localization of Bacillus subtilis sacU(Hy) mutations to two linked genes with similarities to the conserved procaryotic family of two-component signalling systems' JOURNAL OF BACTERIOLOGY vol. 170, no. 11, November 1988, pages 5102 - 5109

## Description

### Field of the Invention

The present invention relates to the field of molecular biology and in particular to the production of proteins in gram-positive microorganisms. In particular, the present invention relates to gram-positive microorganisms having a mutation in the opp operon and methods for producing proteins in such host cells.

### Background

Gram-positive microorganisms, such as Bacillus, have been used for large-scale industrial fermentation due, in part, to their ability to secrete their fermentation products into the culture media. Secreted proteins are exported across a cell membrane and a cell wall, and then are subsequently released into the external media. It is advantageous to produce proteins of interest in gram-positive microorganisms since exported proteins usually maintain their native conformation.

The opp operon of Bacillus (also known in the art as spoOK operon) encodes an oligopeptide permease that is required for the initiation of sporulation and the development of genetic competence (Rudner et al, 1991, Journal of Bacteriology, 173:1388-1398). The opp operon is a member of the family of ATP-binding cassette transporters involved in the import or export of oligopeptides from 3-5 amino acids. There are five gene products of the opp operon: oppA is the ligand-binding protein and is attached to the outside of the cell by a lipid anchor; oppB and oppC are the membrane proteins that form a complex through which the ligand is transported; oppD and oppF (Perego et al., 1991, Mol. Microbiol. 5:173-185) are the ATPases thought to provide energy for transport (LeDeaux et al., 1997, FEMS Microbiology Letters 153: 63-69). The opp operon has also been referred to as SpoOK by Rudner et al., 1991, J. Bacteriol. 173:1388-1398).

Although deletion mutations in the B.subtilis opp operon have been made (LaDeaux, 1997, FEMS Microbiology Letters 153:63-69) these deletions have not been correlated with enhanced expression of recombinant proteins in B. subtilis. There remains a need for improved methods for the production of proteins in Bacillus as well as other gram-positive microorganisms.

### Summary of the Invention

The present invention is based, in part, upon the discovery that a Bacillus strain containing a mutation in the opp operon produces more recombinant protein than the wild-type Bacillus strain. Accordingly, the present invention provides a method for producing a protein in a gram-positive microorganism comprising the steps of obtaining a gram positive microorganism comprising nucleic acid encoding said protein, said microorganism having a mutation in the oppA gene in the opp operon said mutation resulting in the inactivation of the oppA product said opp operon; and culturing said microorganism under conditions suitable for the expression of said protein. In another embodiment, the gram-positive microorganism is a member of the family *Bacillus.* In another embodiment, the Bacillus includes *B. licheniformis, B. lentus, B. brevis, B. stearothermophilus, B. alkalophilus, B. amyloliquefaciens, B. coagulans, B. circulans, B. lautus and Bacillus thuringiensis*.

In a further embodiment of the present invention, the protein includes hormone, enzyme, growth factor and cytokine. In yet another embodiment, the protein is an enzyme and includes proteases, carbohydrases, and lipases; isomerases such as racemases, epimerases, tautomerases, or mutases; transferases, kinases and phophatases. In one aspect of the present invention, the protein is protease obtainable from a Bacillus species. In another aspect of the present invention, the protease is subtilisn.

### Brief Description of the Drawings

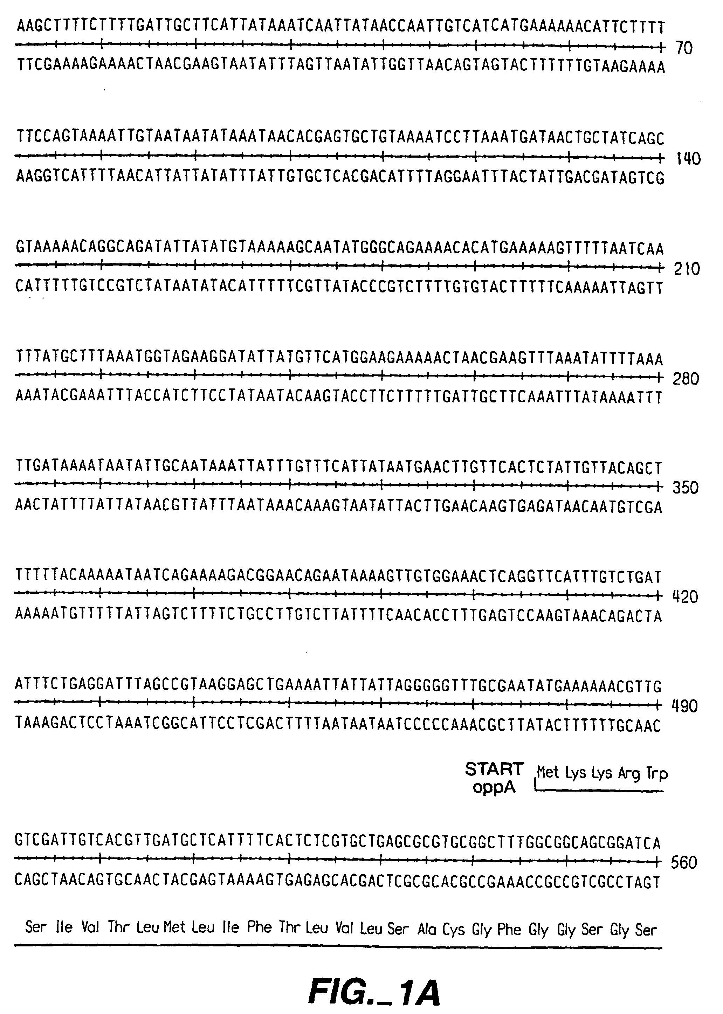

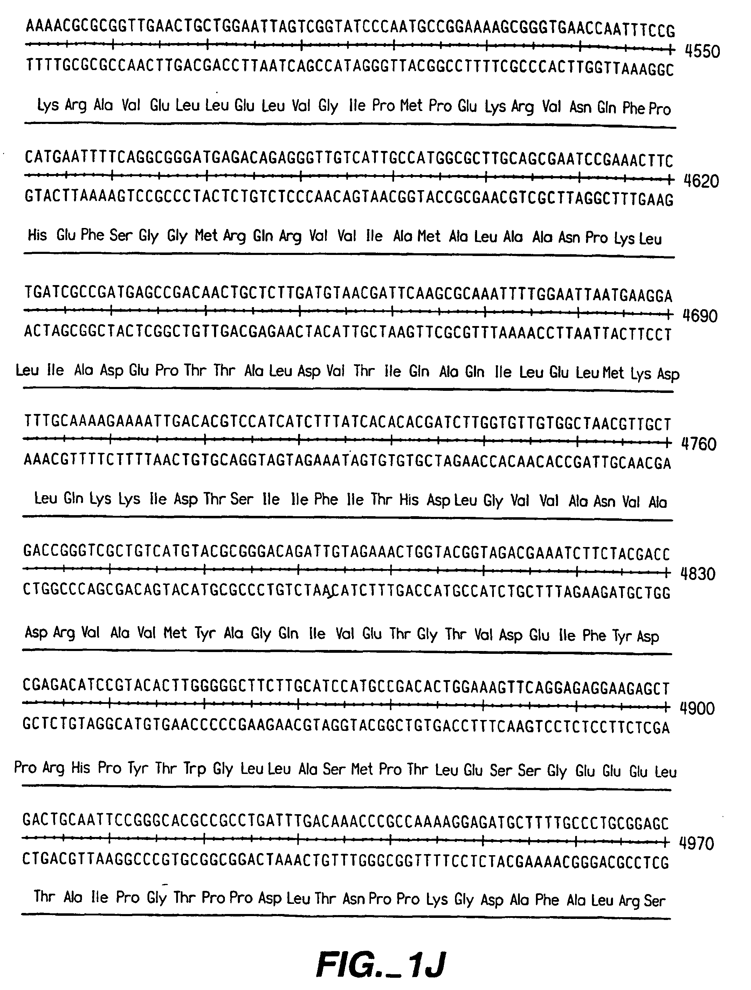

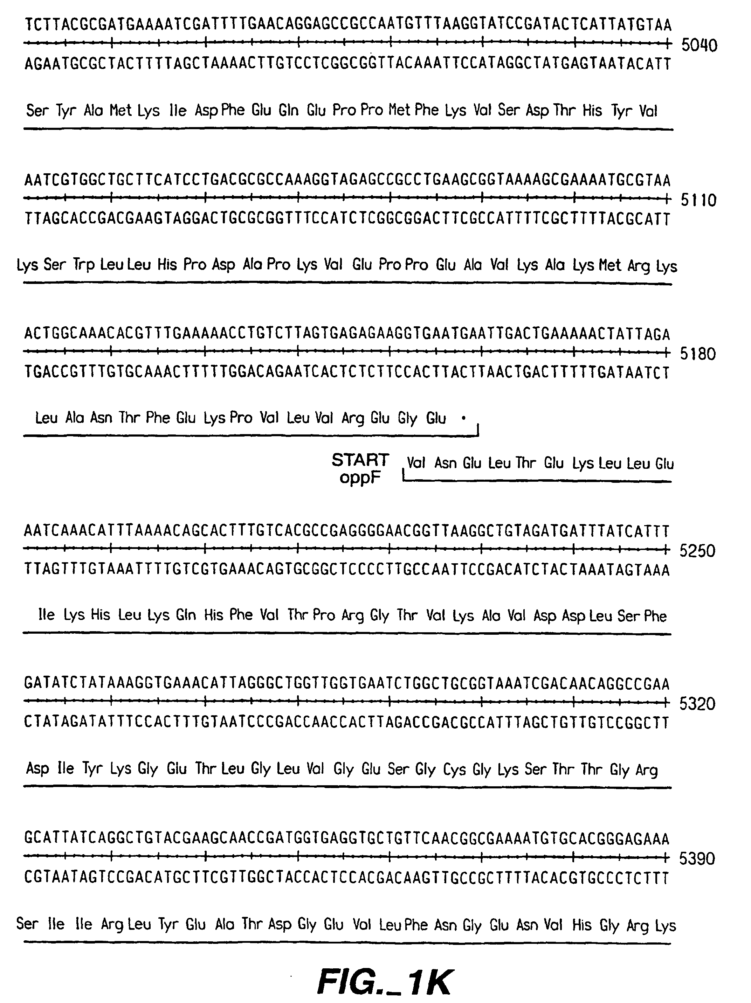

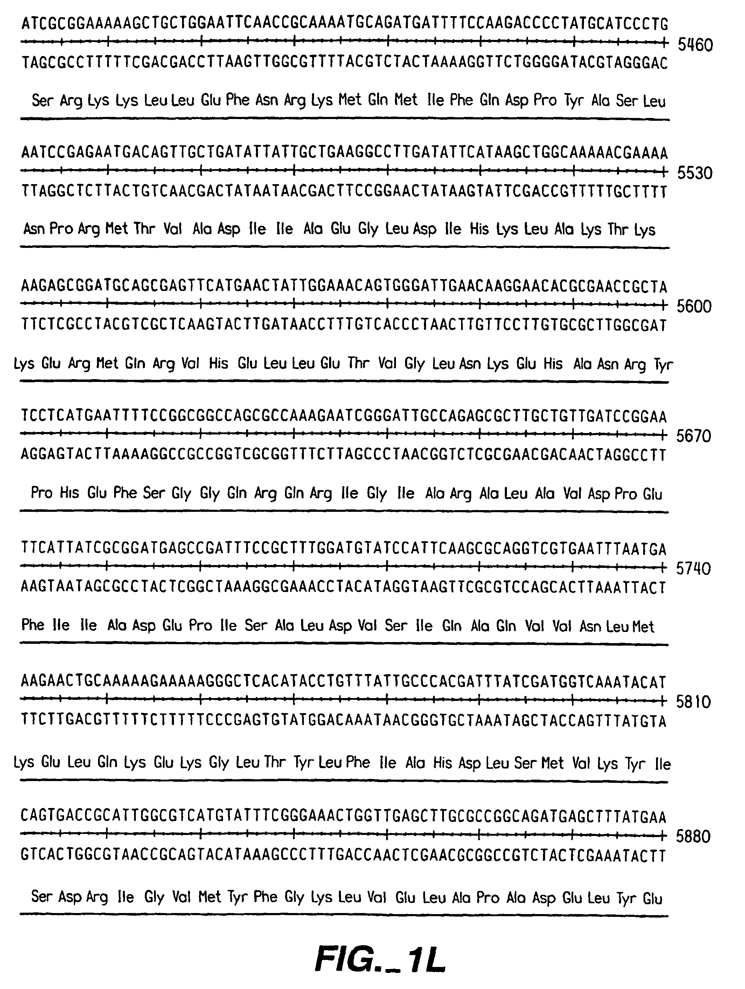

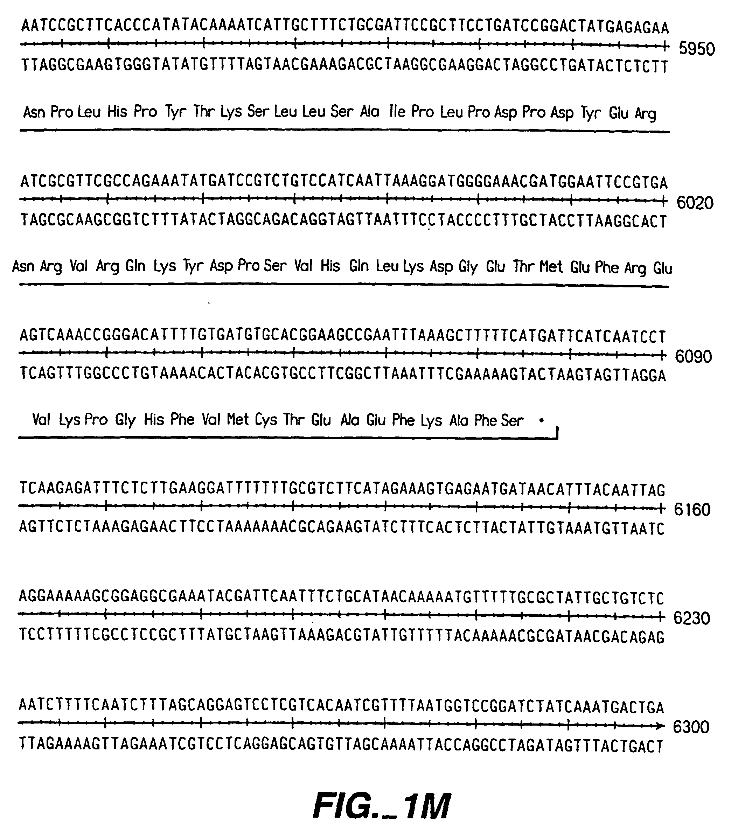

Figures 1A-1M shows the nucleic acid and amino acid sequence of the B.subtilis opp operon. The oppA, oppB, oppC, oppD and oppF genes are designated.

### Detailed Description

### Definitions

As used herein, the genus *Bacillus* includes all members known to those of skill in the art, including but not limited to *B. subtilis, B. licheniformis, B. lentus, B. brevis, B. stearothermophilus, B. alkalophilus, B. amyloliquefaciens, B. coagulans, B. ciculans, B. lautus and B. thuringiensis*.

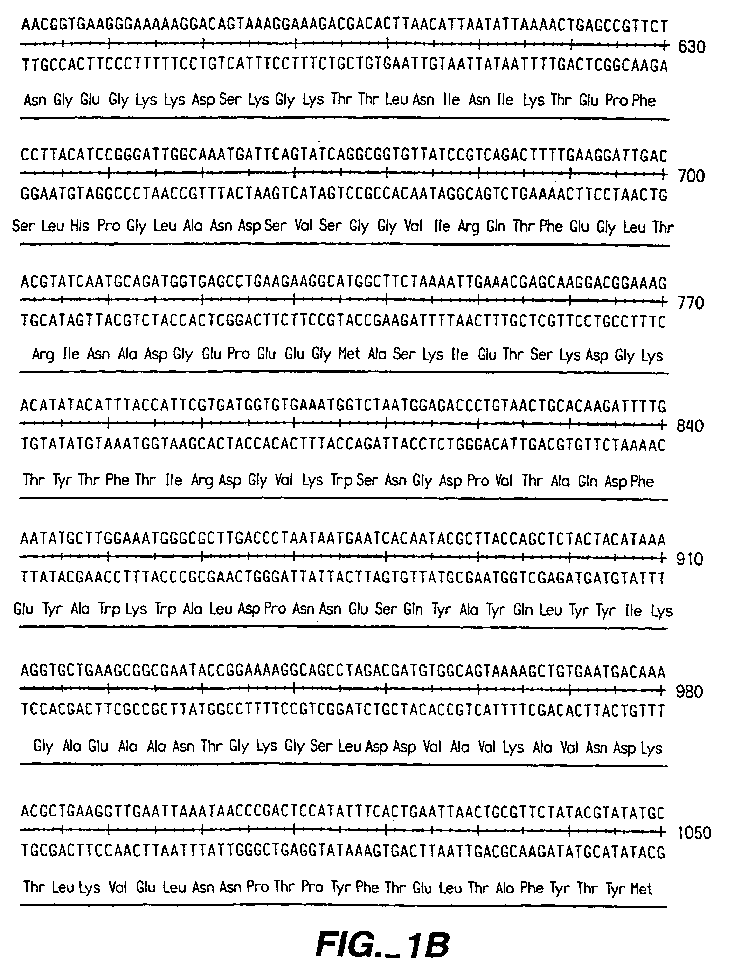

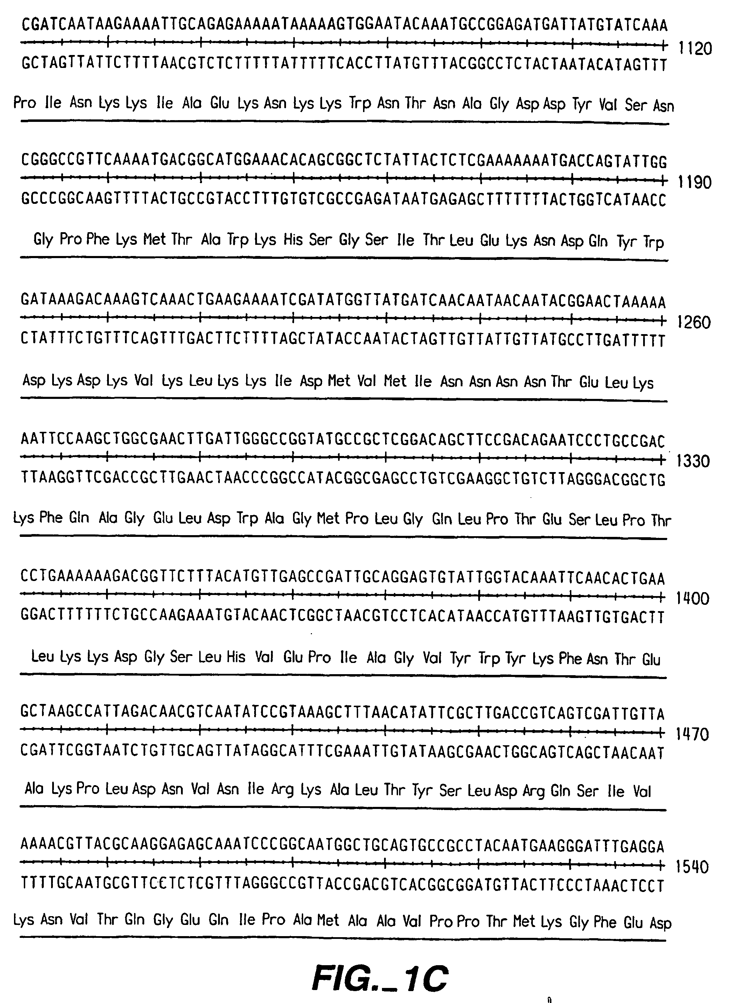

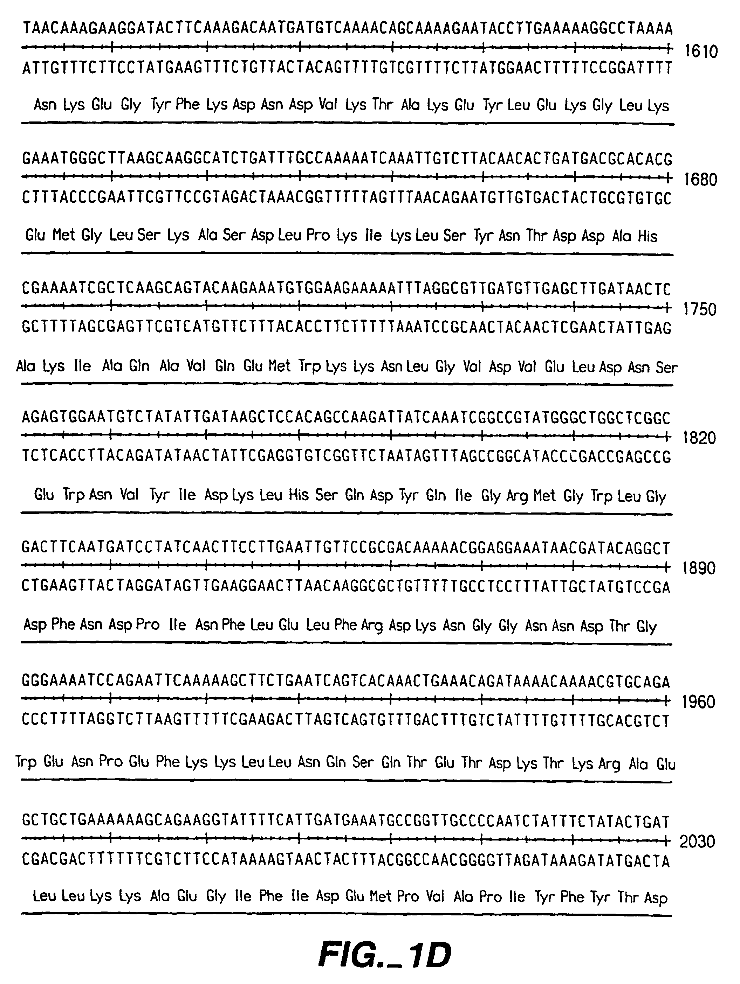

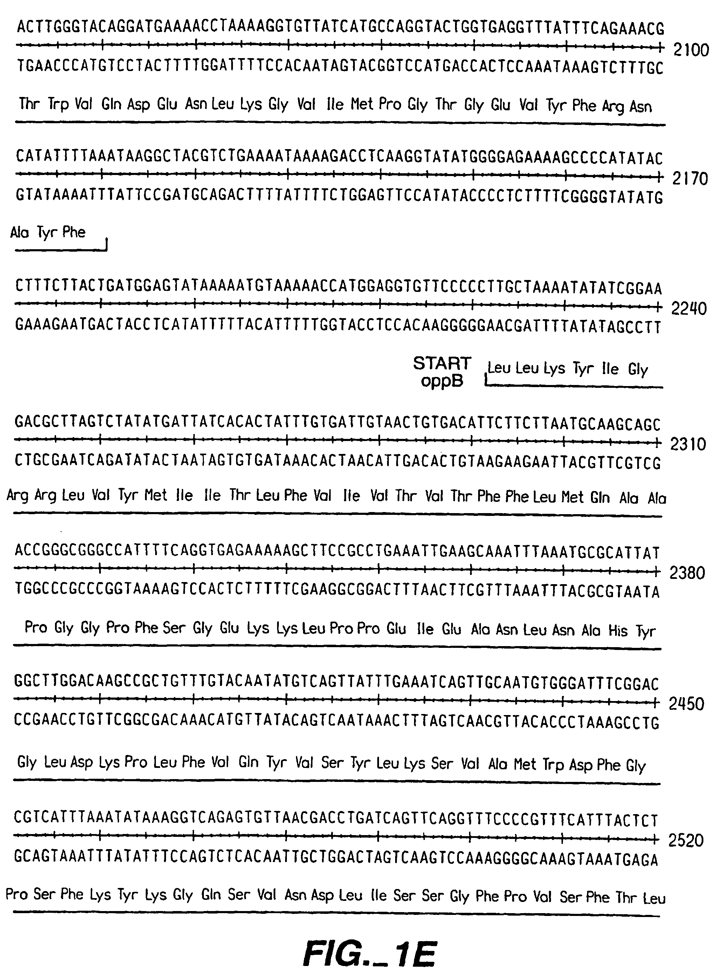

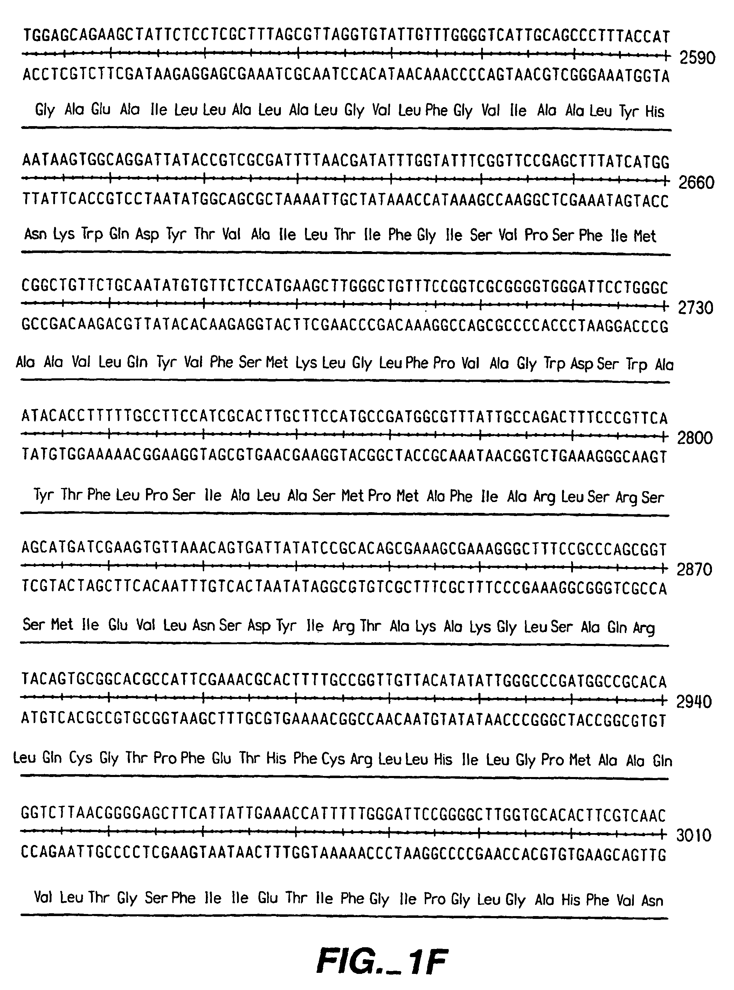

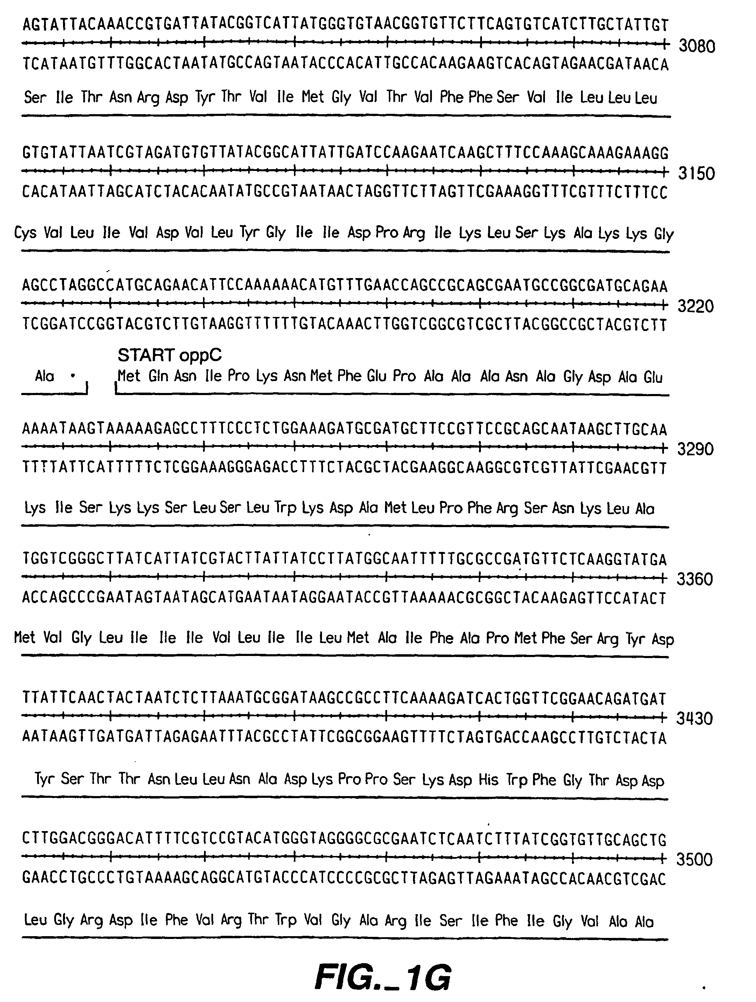

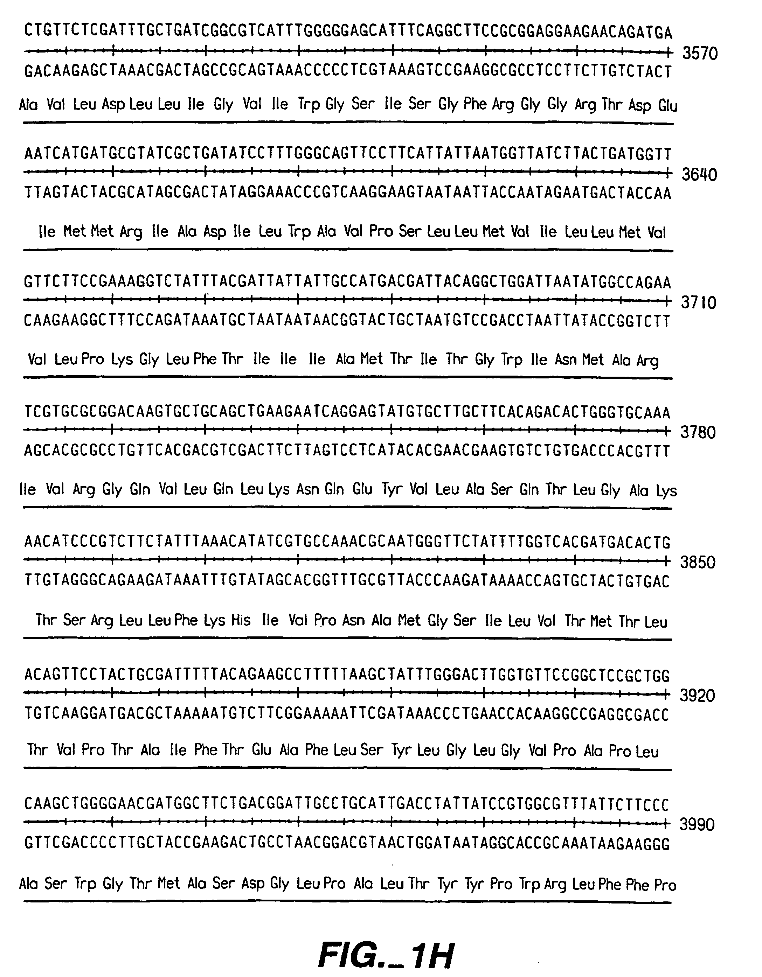

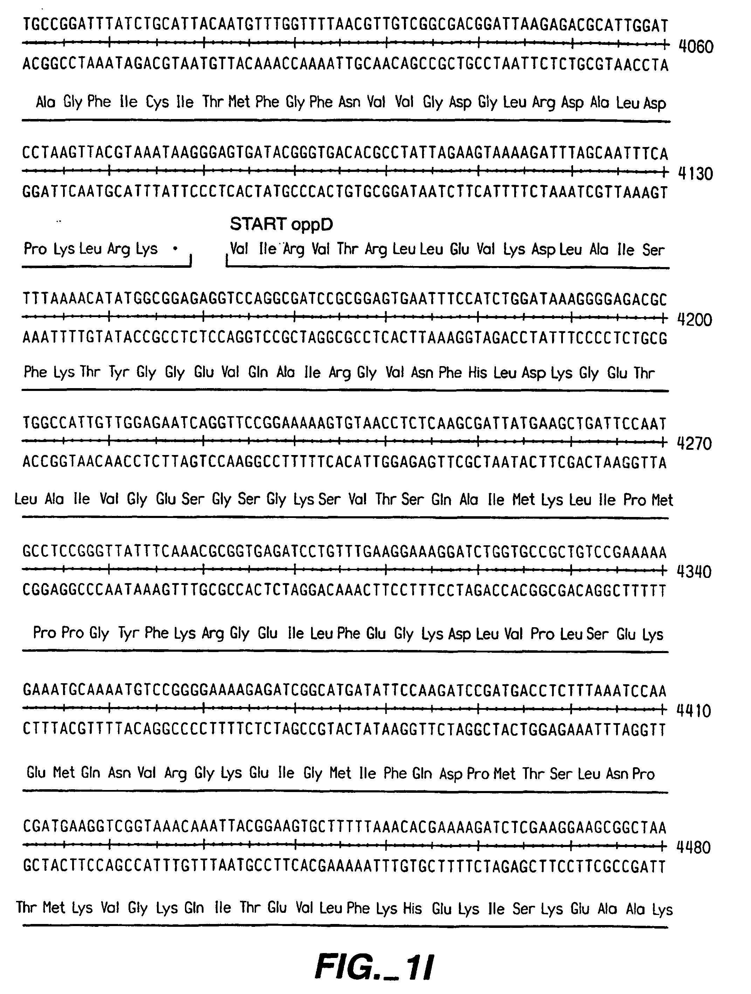

As used herein the term "Bacillus subtilis opp operon" refers to the B. subtilis operon sequence disclosed in Figures 1A-1M, with the individual genes, oppA, oppB, oppC, oppD and oppF, designated. The term "opp operon" encompasses opp operons present in gram positive organisms. A gram positive microorganism may have a cluster of multiple genes comprising the opp operon, similar to B. subtilis. The term opp operon refers to the cluster of genes collectively. Gram positive microorganism opp operons are disclosed in Podbielski et al. (1996, Molecular Microbiology 21: 1087-1099 and Tynkkynen et al. (1993, Journal of Bacteriology 175: 7523-7532). Bacillus opp operons will comprise nucleic acid having at least 50%, at least 55%, at least 60%, at least 65%, at least 70%, at least 75%, at least 80%, at least 85%, at least 90% or at least 95% identity to *B.subtilis* opp operon shown in Figure 1 and will function to import peptides for the Bacillus. Percent identity may be determined over the entire length of the opp operon or may be determined on a gene basis for any individual gene in the opp operon gene cluster.

In one embodiment, the gram-positive organisms is a *Bacillus.* In another embodiment, the gram-positive organism is *B. subtilis, B. licheniformis, B. lentus, B. brevis, B. stearothermophilus, B. alkalophilus, B. amyloliquefaciens, B. coagulans, B. ciculans, B. lautus and B. thuringiensis*. In a preferred embodiment, the gram-positive microorganism is *Bacillus subtilis.*

As used herein, "nucleic acid" refers to a nucleotide or polynucleotide sequence, and fragments or portions thereof, and to DNA or RNA of genomic or synthetic origin which may be double-stranded or single-stranded, whether representing the sense or antisense strand. As used herein "amino acid" refers to peptide or protein sequences or portions thereof.

The terms "isolated" or "purified" as used herein refer to a nucleic acid or amino acid that is removed from at least one component with which it is naturally associated.

As used herein, the term "heterologous protein" refers to a protein or polypeptide that does not naturally occur in a gram-positive host cell. Examples of heterologous proteins include enzymes such as hydrolases including proteases, cellulases, amylases, carbohydrases, and lipases; isomerases such as racemases, epimerases, tautomerases, or mutases; transferases, kinases and phophatases. The heterologous gene may encode therapeutically significant proteins or peptides, such as growth factors, cytokines, ligands, receptors and inhibitors, as well as vaccines and antibodies. The gene may encode commercially important industrial proteins or peptides, such as proteases, carbohydrases such as amylases and glucoamylases, cellulases, oxidases and lipases. The gene of interest may be a naturally occurring gene, a mutated gene or a synthetic gene.

The term "homologous protein" refers to a protein or polypeptide native or naturally occurring in a gram-positive host cell. The invention includes host cells producing the homologous protein via recombinant DNA technology. The present invention encompasses a gram-positive host cell having a deletion or interruption of the nucleic acid encoding the naturally occurring homologous protein, such as a protease, and having nucleic acid encoding the homologous protein re-introduced in a recombinant form. In another embodiment, the host cell produces the homologous protein. A recombinant protein refers to any protein encoded by a nucleic acid which has been introduced into the microorganism.

As used herein, the term "mutation" refers to any alteration in at least one of the genes in the opp operon such that the gene product is inactivated or eliminated and transport of oligopeptides of 3-5 amino acids is diminished or eliminated. Examples of mutations include but are not limited to point mutations, frame shift mutations and deletions of part of all of a gene in the opp operon gene cluster. The term "mutation" include alterations in any or all of the genes in the opp operon gene cluster.

### Detailed Description of the Preferred Embodiments

The present invention is based upon the discovery that mutating at least one gene of the opp operon in a gram-positive microorganism leads to increased production of heterologous proteins in the mutated microorganism. This discovery provides a basis for producing host microorganisms and expression methods which can be used to produce heterologous proteins. In a preferred embodiment, the host cell is a Bacillus species that has a mutation in oppA such that the oppA gene product is inactivated. The Bacillus is further genetically engineered to produced a heterologous or homologous protein or polypeptide. In one embodiment, the heterologous protein includes proteases, carbohydrases such as amylases and glucoamylases, cellulases, oxidases and lipases. In a preferred embodiment, the polypeptide is a protease obtainable from Bacillus species. In another preferred embodiment, the protease is subtilisn. The nucleic acid and amino acid sequences for subtilisn are found in the following publications: *B. subtilis*: Stahl, M. L. and E. Ferrari 1984 J Bacteriol 158, 411-418; *B. amyloliquefaciens*: Wells, J. A., E. Ferrari, D. J. Henner, D. A. Estell, and E. Y. Chen 1983 Nucleic Acid Res 11, 7911-7925; N. Vasantha, L. D. Thompson, C. Rhodes, et al 1984, J. Bacteriol, 159, 811-819; *B. amylosachariticus*: Kurhara M, Markland, F. S. and Smith E. L. 1972, J. Biol. Chem, 247, 5619-5631; *B. lentus*: Hastrup, S., Branner, S., Norris, F., et al 1989 International Patent No. WO 89/0629; *B. licheniformis*: Jacobs, M., Eliasson, M., Uhlen, M., and Flock, J. 1985, Nucleic Acid Res 13, 8913-8927.

### I. Opp operon

The opp operon is known to be associated with the transport, i.e., import of oligopeptides of 3-5 amino acids. The sequence for the Bacillus subtilis opp operon is given in Figures 1A-1M. The present invention encompasses a mutation in at least one of the genes of the opp operon gene cluster such that the gene product is inactivated or eliminated and peptide transport is interrupted. One assay for the presence or absence of a functioning opp operon is to subject the host microorganism to growth in the presence of toxic oligopeptide of 3 amino acids, such as Bialaphos, a tripeptide consiting of two L-alanine molecules and an L-glutamic acid analogue (Meiji Seika, Japan). A microorganism having a functional opp operon will have inhibited growth. A microorganism having a mutation in at least one gene of the opp operon gene cluster will not show growth inhibition in the presence of the toxic oligopeptide.

Gram-positive polynucleotide homologs of *B.subtilis* opp operon may be identified and obtained by standard procedures known in the art from, for example, cloned DNA (*e.g.,* a DNA "library"), genomic DNA libraries, by chemical synthesis once identified, by cDNA cloning, or by the cloning of genomic DNA, or fragments thereof, purified from a desired cell. (*See,* for example, Sambrook *et al*., 1989, Molecular Cloning, A Laboratory Manual, 2d Ed., Cold Spring Harbor Laboratory Press, Cold Spring Harbor, New York; Glover, D.M. (ed.), 1985, DNA Cloning: A Practical Approach, MRL Press, Ltd., Oxford, U.K. Vol. I, II.) The isolated opp operon, or alternatively, individual opp operon genes A, B, C, D, or F, can be molecularly cloned into a suitable vector for propagation. In the molecular cloning of the gene from genomic DNA, DNA fragments are generated, some of which will encode the desired gene. The DNA may be cleaved at specific sites using various restriction enzymes. Alternatively, one may use DNAse in the presence of manganese to fragment the DNA, or the DNA can be physically sheared, as for example, by sonication. The linear DNA fragments can then be separated according to size by standard techniques, including but not limited to, agarose and polyacrylamide gel electrophoresis and column chromatography.

Once the DNA fragments are generated, identification of the specific DNA fragment containing the opp operon may be accomplished in a number of ways. For example, *B.subtilis* oppA gene or its specific RNA, or a fragment thereof, such as a probe or primer, may be isolated and labeled and then used in hybridization assays to detect a gram-positive oppA gene. (Benton, W. and Davis, R., 1977, Science 196:180; Grunstein, M. And Hogness, D., 1975, Proc. Natl. Acad. Sci. USA 72:3961). Those DNA fragments sharing substantial sequence similarity to the probe will hybridize under stringent conditions.

Hybridization conditions are based on the melting temperature (Tm) of the nucleic acid binding complex, as taught in Berger and Kimmel (1987, Guide to Molecular Cloning Techniques, Methods in Enzymology, Vol 152, Academic Press, San Diego CA) incorporated herein by reference, and confer a defined "stringency" as explained below.

"Maximum stringency" typically occurs at about Tm-5°C (5°C below the Tm of the probe); "high stringency" at about 5°C to 10°C below Tm; "intermediate stringency" at about 10°C to 20°C below Tm; and "low stringency" at about 20°C to 25°C below Tm. As will be understood by those of skill in the art, a maximum stringency hybridization can be used to identify or detect identical polynucleotide sequences while an intermediate or low stringency hybridization can be used to identify or detect polynucleotide sequence homologs.

The term "hybridization" as used herein shall include "the process by which a strand of nucleic acid joins with a complementary strand through base pairing" (Coombs J (1994) Dictionary of Biotechnology, Stockton Press, New York NY).

The process of amplification as carried out in polymerase chain reaction (PCR) technologies is described in Dieffenbach CW and GS Dveksler (1995, PCR Primer, a Laboratory Manual, Cold Spring Harbor Press, Plainview NY). A nucleic acid sequence of at least about 10 nucleotides and as many as about 60 nucleotides from *B. subtilis* opp operon genes, preferably about 12 to 30 nucleotides, and more preferably about 20-25 nucleotides can be used as a probe or PCR primer.

### II. Expression Systems

The present invention provides host microorganisms and expression methods for the production and secretion of desired heterologous proteins in gram-positive microorganisms. In one embodiment, the host cell is genetically engineered to have a mutation in at least one gene of the opp operon gene cluster such that the gene product is eliminated or inactivated. In a preferred embodiment, the mutation is a frame shift mutation in the oppA gene. In another embodiment of the present invention, a gram-positive microorganism having a mutation in at least one gene of the opp operon is genetically engineered to further comprise nucleic acid encoding a heterologous or homologous protein.

### Inactivation of genes in the opp operon in a host cell

Producing a gram-positive microorganism incapable of producing at least one gene of the opp operon necessitates inactivating or eliminating the naturally occurring opp operon gene from the genome of the host cell. In a preferred embodiment, the inactivation is the result of a mutation and is preferrably a non-reverting mutation.

One method for mutating nucleic acid encoding a gram-positive microorganism opp operon gene is to clone the nucleic acid or part thereof, modify the nucleic acid by site directed mutagenesis and reintroduce the mutated nucleic acid into the cell on a plasmid. By homologous recombination, the mutated gene may be introduced into the chromosome. In the parent host cell, the result is that the naturally occurring nucleic acid and the mutated nucleic acid are located in tandem on the chromosome. After a second recombination, the modified sequence is left in the chromosome having thereby effectively introduced the mutation into the chromosomal gene for progeny of the parent host cell.

Another method for mutating an opp operon gene is through deleting the chromosomal gene copy. In a preferred embodiment, the entire gene is deleted, the deletion occurring in such as way as to make reversion impossible. In another preferred embodiment, a partial deletion is produced, provided that the nucleic acid sequence left in the chromosome is too short for homologous recombination with a plasmid encoded gene.

Deletion of the naturally occurring gram-positive microorganism opp operon gene can be carried out as follows. An opp gene including its 5' and 3' regions is isolated and inserted into a cloning vector. The coding region of the gene is deleted from the vector *in vitro,* leaving behind a sufficient amount of the 5' and 3' flanking sequences to provide for homologous recombination with the naturally occurring gene in the parent host cell. The vector is then transformed into the gram-positive host cell. The vector integrates into the chromosome via homologous recombination in the flanking regions. This method leads to a gram-positive strain in which the opp gene has been deleted.

The vector used in an integration method is preferably a plasmid. A selectable marker may be included to allow for ease of identification of desired recombinant microorgansims. Additionally, as will be appreciated by one of skill in the art, the vector is preferably one which can be selectively integrated into the chromosome. This can be achieved by introducing an inducible origin of replication, for example, a temperature sensitive origin into the plasmid. By growing the transformants at a temperature to which the origin of replication is sensitive, the replication function of the plasmid is inactivated, thereby providing a means for selection of chromosomal integrants. Integrants may be selected for growth at high temperatures in the presence of the selectable marker, such as an antibiotic. Integration mechanisms are described in WO 88/06623.

Integration by the Campbell-type mechanism can take place in the 5' flanking region of the desired gene, resulting in a strain carrying the entire plasmid vector in the chromosome in the opp locus. Since illegitimate recombination will give different results it will be necessary to determine whether the complete gene has been deleted, such as through nucleic acid sequencing or restriction maps.

Another method of mutating a naturally occurring opp gene is to mutagenize the chromosomal gene copy by transforming a gram-positive microorganism with oligonucleotides which are mutagenic. Alternatively, the chromosomal opp gene can be mutated by replacing the chromosomal gene by a mutant gene by homologous recombination.

The present invention encompasses gram-positive microorganism host cells having additional protease mutations, such as mutations in apr, npr, epr, mpr and others known to those of skill in the art.

### Vector Sequences

For production of proteins in a gram-positive microorganism, an expression vector comprising at least one copy of nucleic acid encoding the heterologous or homologous protein, and preferably comprising multiple copies, is transformed into the cell under conditions suitable for expression of the protein. In a preferred embodiment, the protein is a protease obtainable from a Bacillus species.

Expression vectors used in expressing the heterologous proteins of the present invention in gram-positive microorganisms comprise at least one promoter associated with the protein, which promoter is functional in the host cell. In one embodiment of the present invention, the promoter is the wild-type promoter for the selected protein and in another embodiment of the present invention, the promoter is heterologous to the protein, but still functional in the host cell. In one preferred embodiment of the present invention, nucleic acid encoding the protease is stably integrated into the microorganism genome.

In a preferred embodiment, the expression vector contains a multiple cloning site cassette which preferably comprises at least one restriction endonuclease site unique to the vector, to facilitate ease of nucleic acid manipulation. In a preferred embodiment, the vector also comprises one or more selectable markers. As used herein, the term selectable marker refers to a gene capable of expression in the gram-positive host which allows for ease of selection of those hosts containing the vector. Examples of such selectable markers include but are not limited to antibiotics, such as, erythromycin, actinomycin, chloramphenicol and tetracycline.

### III. Transformation

In a preferred embodiment, the host cell is a gram-positive microorganism and in another preferred embodiment, the host cell is *Bacillus.* In one embodiment of the present invention, nucleic acid encoding at least one heterologous protein is introduced into a host cell via an expression vector capable of replicating within the host cell. Suitable replicating plasmids for *Bacillus* are described in Molecular Biological Methods for *Bacillus,* Ed. Harwood and Cutting, John Wiley & Sons, 1990, hereby expressly incorporated by reference; see chapter 3 on plasmids. Suitable replicating plasmids for *B. subtilis* are listed on page 92. Several strategies have been described in the literature for the direct cloning of DNA in *Bacillus.* Plasmid marker rescue transformation involves the uptake of a donor plasmid by competent cells carrying a partially homologous resident plasmid (Contente *et al*., *Plasmid 2*:555-571 (1979); Haima *et al*., *Mol. Gen. Genet. 223*:185-191 (1990); Weinrauch *et al*., *J. Bacteriol. 154(3)*:1077-1087 (1983); and Weinrauch *et al*., *J. Bacteriol. 169(3)*:1205-1211 (1987)). The incoming donor plasmid recombines with the homologous region of the resident "helper" plasmid in a process that mimics chromosomal transformation.

Transformation by protoplast transformation is described for *B. subtilis* in Chang and Cohen, (1979) Mol. Gen. Genet 168:111-115; for B.megaterium in Vorobjeva et al., (1980) FEMS Microbiol. Letters 7:261-263; for B. amyloliquefaciens in Smith et al., (1986) Appl. and Env. Microbiol. 51:634; for B.thuringiensis in Fisher et al., (1981) Arch. Microbiol. 139:213-217; for B.sphaericus in McDonald (1984) J. Gen. Microbiol. 130:203; and B.larvae in Bakhiet et al., (1985) 49:577. Mann et al., (1986, Current Microbiol. 13:131-135) report on transformation of *Bacillus* protoplasts and Holubova, (1985) Folia Microbiol. 30:97) disclose methods for introducing DNA into protoplasts using DNA containing liposomes. The presence/absence of a marker gene can suggest whether the gene of interest is present in the host microorganism.

Alternatively, host cells which contain the coding sequence for an opp operon gene and express the protein may be identified by a variety of procedures known to those of skill in the art. These procedures include, but are not limited to, DNA-DNA or DNA-RNA hybridization and protein bioassay or immunoassay techniques which include membrane-based, solution-based, or chip-based technologies for the detection and/or quantification of the nucleic acid or protein.

### IV. Assay of Protein Activity

There are various assays known to those of skill in the art for detecting and measuring activity of heterologous proteins or polypeptides. In particular, for proteases, there are assays based upon the release of acid-soluble peptides from casein or hemoglobin measured as absorbance at 280 nm or colorimetrically using the Folin method (Bergmeyer, et al., 1984, Methods of Enzymatic Analysis vol. 5, Peptidases, Proteinases and their Inhibitors, Verlag Chemie, Weinheim). Other assays involve the solubilization of chromogenic substrates (Ward, 1983, Proteinases, in Microbial Enzymes and Biotechnology (W.M. Fogarty, ed.), Applied Science, London, pp. 251-317).

### V. Secretion of Recombinant Proteins

Means for determining the levels of secretion of a heterologous or homologous protein in a gram-positive host cell and detecting secreted proteins include, using either polyclonal or monoclonal antibodies specific for the protein. Examples include enzyme-linked immunosorbent assay (ELISA), radioimmunoassay (RIA) and fluorescent activated cell sorting (FACS). These and other assays are described, among other places, in Hampton R et al (1990, Serological Methods, a Laboratory Manual, APS Press, St Paul MN) and Maddox DE et al (1983, J Exp Med 158:1211).

A wide variety of labels and conjugation techniques are known by those skilled in the art and can be used in various nucleic and amino acid assays. Means for producing labeled hybridization or PCR probes for detecting specific polynucleotide sequences include oligolabeling, nick translation, end-labeling or PCR amplification using a labeled nucleotide. Alternatively, the nucleotide sequence, or any portion of it, may be cloned into a vector for the production of an mRNA probe. Such vectors are known in the art, are commercially available, and may be used to synthesize RNA probes *in vitro* by addition of an appropriate RNA polymerase such as T7, T3 or SP6 and labeled nucleotides.

A number of companies such as Pharmacia Biotech (Piscataway NJ), Promega (Madison WI), and US Biochemical Corp (Cleveland OH) supply commercial kits and protocols for these procedures. Suitable reporter molecules or labels include those radionuclides, enzymes, fluorescent, chemiluminescent, or chromogenic agents as well as substrates, cofactors, inhibitors, magnetic particles and the like. Patents teaching the use of such labels include US Patents 3,817,837; 3,850,752; 3,939,350; 3,996,345; 4,277,437; 4,275,149 and 4,366,241. Also, recombinant immunoglobulins may be produced as shown in US Patent No. 4,816,567 and incorporated herein by reference.

### VI. Purification of Proteins

Gram positive host cells transformed with polynucleotide sequences encoding heterologous or homologous protein may be cultured under conditions suitable for the expression and recovery of the encoded protein from cell culture. The protein produced by a recombinant gram-positive host cell comprising a protease of the present invention will be secreted into the culture media. Other recombinant constructions may join the heterologous or homologous polynucleotide sequences to nucleotide sequence encoding a polypeptide domain which will facilitate purification of soluble proteins (Kroll DJ et al (1993) DNA Cell Biol 12:441-53).

Such purification facilitating domains include, but are not limited to, metal chelating peptides such as histidine-tryptophan modules that allow purification on immobilized metals (Porath J (1992) Protein Expr Purif 3:263-281), protein A domains that allow purification on immobilized immunoglobulin, and the domain utilized in the FLAGS extension/affinity purification system (Immunex Corp, Seattle WA). The inclusion of a cleavable linker sequence such as Factor XA or enterokinase (Invitrogen, San Diego CA) between the purification domain and the heterologous protein can be used to facilitate purification.

### VII. Uses of The Present Invention

The present invention provides genetically engineered gram-positive host microorganisms comprising preferably non-revertable mutations in at least one gene of the opp operon gene cluster such that the activity of the gene product is inactivated or eliminated and the transport mechanism is interrupted. The host microorganism may contain additional protease deletions, such as deletions of the mature subtilisn protease and/or mature neutral protease disclosed in United States Patent No. 5,264,366.

In a preferred embodiment, the microorganism is genetically engineered to produce a desired protein or polypeptide. In a preferred embodiment the gram positive microorganism is a *Bacillus* and the polypeptide produced is a protease obtainable from a Bacillus species. In an illustrative embodiment disclosed herein, the protease is subtilisn. In another embodiment disclosed herein, the protein is amylase.

The manner and method of carrying out the present invention may be more fully understood by those of skill in the art by reference to the following examples, which examples are not intended in any manner to limit the scope of the present invention or of the claims directed thereto

### Example I

Example I illustrates the increase in production of subtilisn from B. subtilis having a mutation in the oppA gene of the opp operon.

Bacillus subtilis was cultured in the presence of the toxic peptide Bialaphos (50 µg/ml), a tripeptide consiting of two L-alanine molecules and an L-glutamic acid analogue and was shown to have growth inhibition. Bacillus subtilis comprising nucleic acid encoding subtilisn obtainable from B.subtilis and having a mutation in the degU gene (United States Patent No. 5,387,521) did not show inhibition of growth growing on a plate containing Bialaphos (50 µg/ml). This B. subtilis strain was subjected to PCR using the following primers:
Primer 1 GTTGTGGAAACTCAGGTTCATTTGTC and Primer 2 GGCCCGCCCGGTGCTGCTTGC. The PCR fragment generated using the primers was sequenced and found to have a T-insertion at the beginning of the oppA gene.

A plasmid containing a fragment from the oppA wild-type gene was constructed using PCR technique. 857 bp of the *oppA* gene present in Bacillus subtilis was amplified with the addition of restriction sites using the following primers:
GCGCGCGGATCCCCTTAAATGATAACTGCTATCAGCGTAAAAACAGGC, introducing BamHI and GCGCGCCTGCAGCACAGCTTTTACTGCCACATCGTCTAGGCTGCC, introducing PstI.

The amplified DNA was cloned in pTSpUCKan plasmid after digesting the plasmid with BamHI and PstI yielding plasmid Pm100. Plasmid pTSpUCKan carries a Kanamycin resistance gene (Kan') and a temperature sensitive origin of replication (TsOri). The Kan' gene was isolated from pJH1, a *Streptococcus faecalis* plasmid (Trieu-Coet, P. and P. Courvalin. 1983. *Gene* 23: 331-341) as a 1.5 kb ClaI fragment. The ClaI fragment was blunt-ended and cloned into the EcoRV site of plasmid pBluescript II KS (Stratagene) to make plasmid pJM114. Plasmid pJM114 was digested with EcoRI and ClaI and the resulting fragment of 1.489 kb was blunt ended, purified and cloned into the Ndel site of plasmid pUC19 (Yanisch-Perron, C., Vieira, J. and Messing, J. (1985) Gene 33, 103-119) previously digested with Ndel/blunt -ended and phosphatased yielding plasmid pUS19/Kan. The TsOri was obtained from plasmid pE194 (Villafane, R., Bechhofer, D. H., Narayanan, C. S. and Dubnau, D. (1987), J. Bacteriol. 169: 4822-9; Lovet, P. S. and Ambulus, N. P. Jr (1989) Genetic manipulation of Bacillus subtilis. In Biotechnology Handbooks, Vol 2 (Harwood, C. R., ed.), pp. 115-54. Plenun Press: New York and London) by digestion with the restriction enzyme HinPI. The 1.1 kb fragment was blunt-ended and cloned in the HincII site of plasmid pUC19. This new plasmid, Ts-pUC was digested with Xbal and PstI and the 1.163 Kb fragment was blunt ended, purified and cloned into the blunt-ended AatII site in plasmid pUC19Kan, yielding the final plasmid pTSpUCKan.

The oppA mutant in the B.subtilis strain comprising nucleic acid encoding subtilisn and having a mutation in degU was replaced with the oppA wild-type gene using plasmid pM100. Plasmid pM100 was transformed using standard protoplast transformation techniques (Prapai et al., 1994, Microbiology 140:305) into the B. subtilis strain. Because of the TsOri, this plasmid integrated into the chromosome at the region of homology with the oppA gene when cultured under selective pressure at the non-permissive temperature, e.g. 48°C. After integration, the strain carrying the integrated plasmid was grown extensively at permissive temperature. Upon excision of the integrated plasmid, either the parent strain is restored, or a strain carrying the wild-type gene is constructed. A transformant comprising the wild-type oppA gene was confirmed by nucleic acid sequencing by showing inhibition of growth on Bialaphos.

### Example II

Example II illustrates production of subtilisin from strains containing an oppA wild type and an oppA mutant in shake flasks.

A B.subtilis strain comprising nucleic acid encoding B.subtilis subtilisin, a degU mutation and containing wild-type oppA and a strain comprising nucleic acid encoding B.subtilis subtilisin, a degU mutation and containing an oppA mutant produced as in Example I were grown in shake flasks containing 25 ml of LB (Difco) plus 25 ug/mL Chloramphenicol in a 250 mL flask. The shake flasks were incubated at 37°C with vigorous shaking to OD 550 of 0.8. 1 mL of each culture was mixed with 0.5 ml 30 % glycerol and frozen for further experiments. 30 ul of the thawed vials were used to inoculate 40 ml of a media containing 68 g/L Soytone, 300 M PIPES, 20 g/L Glucose (final pH 6.8) in 250 mL flasks. The shake flasks were incubated at 37°C with vigorous shaking for three days, after which aliquots were taken for subtilisin analysis of the supernatant. Results show that the B.subtilis strain containing the oppA mutation produced 19% more subtilisin than when the oppA wild-type gene was present (Table 1).

For the protease assay, supematants from liquid cultures were harvested after 3 days of growth and assayed for subtilisin as previously described (Estell, D. et al (1985) *J. Biol. Chem.* 260, 6518-6521) in a solution containing 0.3 mM *N*-succinyl-L-Ala-L-Ala-L-Pro-L-Phe-*p*-nitroanalide (Vega Biochemicals), 0.1 M Tris, pH 8.6, at 25°C. The assays measured the increase in absorbance at 410 nm/min due to hydrolysis and release of p-nitroanaline.

Table 1 describes the yields of protease produced from the two strains tested.

**Table 1**

| B. subtilis strain genotype | Subtilisin (g/L) | g/L:OD |
|---|---|---|
| oppA- | 1.716 | 18.45 |
| oppAwt | 1.39 | 9.5 |

### Example III

B.subtilis host cells having a T-insertion at the beginning of the oppA gene as described in Example I, were grown in shake flasks containing 25 ml of LB (Difco) in a 250 mL flask. Shake flasks were incubated at 37°C with vigorous shaking and at OD 550 of 0.8, 1 mL of culture was mixed with 0.5 ml 30 % Glycerol and frozen for further experiments. 30 ul of the thawed vials were used to inoculate 40 ml of a media containing 68 g/L Soytone, 300 M PIPES, 20 g/L Glucose (final pH 6.8) in 250 mL flasks. The shake flasks were incubated at 37°C with vigorous shaking for several days during which they were sampled for endogenous amylase activity in the supernatant. Results showed that the strain containing the oppA mutation produced 2 times more B.subtilis endogenous amylase at 48 hours than when the oppA wild-type gene, ie, the wild-type B.subtilis, was present. At 72 hours the increase is 2.4 times, as shown in Table II.

### The Amylase Assay

For the amylase assay, whole broth samples were spun down at different times of growth and their supematants were assayed as follows. 10 ul of the sample was mixed in a cuvete with 790.0 ul of substrate (Megazyme-Ceralpha-Alpha Amylase; substrate is diluted in water and is used as 1 part substrate plus 3 parts of Alpha Amylase buffer pH 6.6) at 25°C. Alpha Amylase buffer is composed of 50mM Maleate Buffer, 5 mM CaCl², and 0.002 % Triton X-100, PH= 6.7. Amylase was measured in a Spectronic Genesys 2 Spectophotometer using a protocol for amylase activity (Wavelenth: 410 nm, Initial Delay: 75 secs., Total Run Time: 120 secs, Lower Limit: 0.08, Upper Limit: 0.12).

Table 2 describes the yields of amylase produced from the two strains tested.

**Table II**

| ***B. subtilis*** **strains** | **Genotype** | **Amylase (rate) 48 h** | **60 h** |
|---|---|---|---|
| 2790 | oppAwt | 0.054 | 0.066 |
| 2790 | oppA- | 0.106 | 0.156 |

## Claims

1. A method for producing a protein or polypeptide in a gram-positive microorganism comprising the steps of, a) obtaining a gram positive microorganism comprising nucleic acid encoding said protein or polypeptide, said microorganism having a mutation in the oppA gene in the opp operon said mutation resulting in the inactivation of the oppA product; and b) culturing said microorganism under conditions suitable for the expression of said protein.

2. The method of Claim 1 wherein said gram-positive microorganism is a member of the family *Bacillus*.

3. The method of Claim 2 wherein said member of the family Bacillus includes *B*. *licheniformis, B. lentus, B. brevis, B. stearothermophilus, B. alkalophilus, B. amyloliquefaciens, B. coagulans, B. circulans, B. lautus and Bacillus thuringiensis*.

4. The method of claim 2 wherein said member of the family Bacillus is B. subtilis.

5. The method of claim 4 wherein B. subtilis has a mutation in the degU gene.

6. The method of Claim 3 wherein said protein includes hormone, enzyme, growth factor and cytokine.

7. The method of Claim 6 wherein said protein is an enzyme.

8. The method of Claim 7 wherein said enzyme includes proteases, carbohydrases, and lipases; isomerases such as racemases, epimerases, tautomerases, or mutases; transferases, kinases and phophatases.

9. The method of Claim 8 wherein said protease is a bacillus protease.

10. The method of Claim 9 wherein said protease is subtilisin.

11. The method of Claim 1 wherein said mutation is non-revertable.

12. The method of Claim 1 wherein said mutation is a frameshift mutation.

13. The method of Claim 8 wherein said carbohydrase is an amylase.

## Patentansprüche

1. Verfahren zur Herstellung eines Proteins oder Polypeptids in einem grampositiven Mikroorganismus, welches die Schritte umfasst, a) einen grampositiven Mikroorganismus zu erhalten, welcher Nukleinsäure, welche das Protein oder Polypeptid kodiert, umfasst, wobei der Mikroorganismus eine Mutation in dem oppA-Gen in dem opp-Operon aufweist, wobei die Mutation zu der Inaktivierung des oppA-Produkts führt; und b) den Mikroorganismus unter Bedingungen, die für die Expression des Proteins geeignet sind, zu kultivieren.

2. Verfahren nach Anspruch 1, wobei der grampositive Mikroorganismus ein Mitglied der Familie *Bacillus* ist.

3. Verfahren nach Anspruch 2, wobei das Mitglied der Famille Bacillus *B. licheniformis, B. lentus, B. brevis, B. stearothermophilus, B. alkalophilus, B. amyloliquefaciens, B.* coagu*lans, B. circulans, B. lautus* und *Bacillus thuringiensis* umfasst.

4. Verfahren nach Anspruch 2, wobei das Mitglied der Familie Bacillus *B. subtilis* ist.

5. Verfahren nach Anspruch 4, wobei *B. subtilis* eine Mutation in dem degU-Gen aufweist.

6. Verfahren nach Anspruch 3, wobei das Protein ein Hormon, ein Enzym, einen Wachstumsfaktor und ein Zytokin umfasst.

7. Verfahren nach Anspruch 6, wobei das Protein ein Enzym ist.

8. Verfahren nach Anspruch 7, wobei das Enzym Proteasen, Carbohydrasen und Upasen; Isomerasen, wie Racemasen, Epimerasen, Tautomerasen oder Mutasen; Transferasen, Kinasen und Phosphatasen umfasst.

9. Verfahren nach Anspruch 8, wobei die Protease eine Bacillus-Protease ist.

10. Verfahren nach Anspruch 9, wobei die Protease Subtillsin ist

11. Verfahren nach Anspruch 1, wobei die Mutation nicht umkehrbar ist.

12. Verfahren nach Anspruch 1, wobei die Mutation eine Leserasterverschiebungsmutation ist.

13. Verfahren nach Anspruch 8, wobei die Carbohydrase eine Amylase ist.

## Revendications

1. Procédé de production d'une protéine dans un micro-organisme gram-positif comprenant les étapes consistant à a) obtenir un micro-organisme gram-positif comportant un acide nucléique codant ladite protéine ou ledit polypeptide, ledit micro-organisme présentant une mutation dans le gène oppA dans l'opéron opp, ladite mutation résultant de l'inactivation du produit oppA dudit opéron opp ; et b) la culture dudit micro-organisme dans des conditions appropriées à l'expression de ladite protéine.

2. Procédé selon la revendication 1, dans lequel le micro-organisme gram-positif est un élément de la famille *Bacillus.*

3. Procédé selon la revendication 2, dans lequel le Bacillus inclut *B. licheniformis, B. lentus, B. brevis, B. stearothermophilus, B. alkalophilus, B. amyloliquefaciens, B. coagulans, B. circulans, B. lautus et Bacillus thuringiensis*.

4. Procédé selon la revendication 2, dans lequel le élément de la famille *Bacillus* est B. subtilis.

5. Procédé selon la revendication 4, dans lequel B. subtilis a une mutation dans le gène degU.

6. Procédé selon la revendication 3, dans lequel ladite protéine inclut une hormone, une enzyme, un facteur de croissance et la cytokine.

7. Procédé selon la revendication 6, dans lequel ladite protéine est une enzyme.

8. Procédé selon la revendication 7, dans lequel ladite enzyme inclut des protéases, des carbohydrases et des lipases ; des isomérases telles que des racémases, des épimérases, des tautomérases, ou des mutases ; des transférases, des kinases et des phosphatases.

9. Procédé selon la revendication 8, dans lequel la protéine est une protéases.

10. Procédé selon la revendication 9, dans lequel la protéase est la subtilisine.

11. Procédé selon la revendication 1, dans lequel ladite mutation est non réversible.

12. Procédé selon la revendication 1, dans lequel ladite mutation est une mutation du cadre de lecture.

13. Procédé selon la revendication 8, dans lequel ladite carbohydrase est une amylase.
